(19) Europäisches Patentamt
European Patent Office
Office européen des brevets

(11) Numéro de publication : **0 368 717 B1**

# (12) FASCICULE DE BREVET EUROPEEN

(45) Date de publication du fascicule du brevet : **20.01.93 Bulletin 93/03**

(51) Int. Cl.$^5$ : **A61K 7/42,** C07D 307/58, C07D 307/94

(21) Numéro de dépôt : **89402970.1**

(22) Date de dépôt : **27.10.89**

## (54) Dérivés de la 5-benzylidène 3-oxa cyclopentanone, leur procédé de préparation et leur utilisation dans des compositions cosmétiques pour la protection de la peau et des cheveux contre le rayonnement solaire.

(30) Priorité : **28.10.88 FR 8814203**

(43) Date de publication de la demande : **16.05.90 Bulletin 90/20**

(45) Mention de la délivrance du brevet : **20.01.93 Bulletin 93/03**

(84) Etats contractants désignés : **BE CH DE GB IT LI**

(56) Documents cités :
**EP-A- 0 044 970**
**EP-A- 0 055 976**
**EP-A- 0 057 882**
**FR-A- 2 395 023**
**CHEMICAL ABSTRACTS, Band 88, 1978, Seite 608, Zusammenfassung Nr. 152593a, Columbus, Ohio, US; & JP-A-77 153 994 (SANKYOCO., LTD.) 21-12-1977**

(73) Titulaire : **L'OREAL**
**14, Rue Royale**
**F-75008 Paris (FR)**

(72) Inventeur : **Lagrange, Alain**
**29, rue Auguste Renoir**
**F-78400 Chatou (FR)**
Inventeur : **Forestier,Serge**
**17, Allée Ferdinand Buisson**
**F-77410 Claye-Souilly (FR)**
Inventeur : **Lang, Gérard**
**44, avenue Lacour**
**F-95210 Saint-Gratien (FR)**
Inventeur : **Luppi, Bernadette**
**43, rue Berlioz**
**F-92270 Sevran (FR)**

(74) Mandataire : **Casalonga, Alain et al**
**BUREAU D.A. CASALONGA - JOSSE**
**Morassistrasse 8**
**W-8000 München 5 (DE)**

**EP 0 368 717 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention est relative à des compositions cosmétiques filtrantes, à leur utilisation pour la protection de la peau et des cheveux contre les rayons ultraviolets, aux nouveaux dérivés de la 5-benzylidène 3-oxa cyclopentanone utilisés dans ces compositions et à leur procédé de préparation.

On sait que les radiations lumineuses de longueurs d'onde comprises entre 280 nm et 400 nm permettent le brunissement de l'épiderme humain et que les rayons de longueurs d'onde comprises entre 280 et 320 nm, connus sous la dénomination d'UV-B, provoquent des érythèmes et des brûlures cutanées qui peuvent nuire au développement du bronzage; ce rayonnement UV-B doit donc être filtré.

On connaît, par le document EP-A- 0 044 970, des 2-(4-hydroxybenzylidène)-gamma-butyrolactones éthérifiées sur le groupe hydroxy phénolique, à titre de filtres solaires UV-B.

On sait également que les rayons UV-A, de longueurs d'onde comprises entre 320 et 400 nm, provoquant le brunissement de la peau, sont susceptibles d'induire une altération de celle-ci notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions phototoxiques ou photo-allergiques.

Il est donc intéressant de disposer de composés susceptibles d'absorber aussi bien les rayons UV-A que les rayons UV-B nocifs pour la peau.

Il est également souhaitable d'assurer aux cheveux une bonne protection contre la dégradation photochimique afin d'éviter un changement de nuance, une décoloration ou une dégradation de leurs propriétés mécaniques.

On sait par ailleurs que les constituants entrant dans les préparations cosmétiques ne possèdent pas toujours une stabilité suffisante à la lumière et se dégradent sous l'action des radiations lumineuses.

Par conséquent, il est souhaitable d'incorporer à ces préparations des composés susceptibles de filtrer les rayons UV et qui doivent présenter en outre une bonne stabilité et une solubilité suffisante dans les milieux habituellement utilisés en cosmétique.

Au cours de ses recherches, la demanderesse a découvert de manière surprenante que certains dérivés de la 5-benzylidène 3-oxa cyclopentanone présentaient, outre de bonnes propriétés filtrantes dans la gamme de longueurs d'onde allant de 280 à 380 nm, une bonne stabilité chimique et photochimique. Ces composés présentent également l'avantage de ne pas être toxiques ou irritants et d'avoir une parfaite innocuité vis-à-vis de la peau. Les composés selon l'invention, hydrosolubles ou liposolubles, présentent également une bonne solubilité dans les solvants cosmétiques usuels.

La présente invention a donc pour objet une composition cosmétique comprenant, dans un support cosmétiquement acceptable, à titre d'agent filtrant le rayonnement ultraviolet de longueurs d'onde comprises entre 280 et 380 nm, une quantité efficace d'au moins un dérivé de 5-benzylidène 3-oxa cyclopentanone ayant la formule générale suivante :

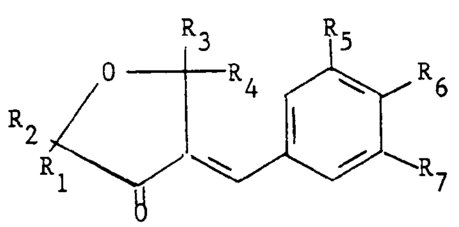

(I)

dans laquelle :

$R_1$, $R_2$, $R_3$ et $R_4$, identiques ou différents, représentent un reste alkyle linéaire ou ramifié en $C_1$-$C_8$, un reste aralkyle tel que benzyle, non substitué ou substitué par des atomes d'halogène ou par des groupes alkyle ou alcoxy en $C_1$-$C_4$, ou un reste aryle tel que phényle, non substitué ou substitué par des atomes d'halogène ou par des restes alkyle ou alcoxy en $C_1$-$C_4$, ou bien $R_1$ et $R_2$ et/ou $R_3$ et $R_4$ forment avec l'atome de carbone auquel ils sont rattachés, un cycle saturé contenant 5 ou 6 atomes de carbone;

$R_5$, $R_6$ et $R_7$, identiques ou différents, représentent un atome d'hydrogène, un reste alkyle linéaire ou ramifié en $C_1$-$C_8$, un reste alcényle linéaire ou ramifié en $C_2$-$C_8$, un reste alcoxy linéaire ou ramifié en $C_1$-$C_{12}$, un reste alcényloxy linéaire ou ramifié en $C_2$-$C_8$, un reste acyloxy linéaire ou ramifié en $C_2$-$C_{12}$, un reste benzyloxy non substitué ou substitué par des atomes d'halogène ou par des restes alkyle ou alcoxy en $C_1$-$C_4$, ou

encore un reste $-COOR_{10}$ où $R_{10}$ désigne un atome d'hydrogène, un reste alkyle linéaire ou ramifié en $C_1$-$C_8$ ou un métal alcalin, alcalino-terreux ou un reste dérivé d'amine; l'un des substituants $R_5$, $R_5$ et $R_7$ peut également représenter un radical de formule :

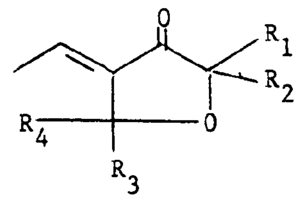

dans lequel $R_1$, $R_2$, $R_3$ et $R_4$ ont les significations mentionnées ci-dessus.

Il est bien entendu que les composés de formule (I) ci-dessus peuvent donner lieu à l'isomérie "cis-trans" autour d'une ou plusieurs double(s) liaison(s) et que tous les isomères font partie de l'invention.

Parmi les composés préférés de formule générale (I) utilisés dans la composition cosmétique filtrante selon l'invention, on peut citer :

- la 5-benzylidène 3-oxa 2,2,4,4-tétraméthyl cyclopentanone,
- la 5-(4'-hexyloxy benzylidène) 3-oxa 2,2,4,4-tétraméthylcyclopentanone,
- la 5-(4'-allyloxy benzylidène) 3-oxa 2,2,4,4-tétraméthyl cyclopentanone,
- la 5-(4'-octyloxy benzylidène) 3-oxa 2,2,4,4-tétraméthyl cylopentanone,
- la 5-(3',4',5'-tribenzyloxy benzylidène) 3-oxa 2,2,4,4-tétraméthyl cyclopentanone,
- la 5-(4'-tert.-butoxy benzylidène) 3-oxa 2,2,4,4-tétraméthyl cyclopentanone,
- la 5-(4'-méthoxycarbonyl benzylidène) 3-oxa 2,2,4,4- tétraméthyl cyclopentanone,
- la 5-(4'-n-butoxy 5'-méthoxy benzylidène) 3-oxa 2,2,4,4-tétraméthyl cyclopentanone,
- le 1,4-bis-[(2',2',4',4'-tétraméthyl-3'-oxa-5'-oxo-1'-cyclopentylidène)méthyl] benzène,
- l'acide 4-[(2',2',4',4'-tétraméthyl-3'-oxa-5'-oxo-1'-cyclopentylidène)méthyl] benzoïque.

Les composés (I) sont solubles dans les huiles sauf dans le cas où l'un des radicaux $R_5$, $R_6$ ou $R_7$ désigne $-COOR_{10}$ où $R_{10}$ désigne un atome d'hydrogène ou un métal alcalin ou alcalino-terreux.

Selon la nature des substituants $R_5$, $R_5$ et $R_7$, les composés de formule (I) ont des maxima d'absorption dans la gamme des UV-B (280-320 nm) ou dans la gamme des UV-A (320-380 nm).

La présente invention a également pour objet un procédé de protection de la peau et des cheveux naturels ou sensibilisés vis-à-vis du rayonnement solaire, consistant à appliquer sur la peau ou les cheveux une quantité efficace d'une composition cosmétique contenant au moins un dérivé de 5-benzylidène 3-oxa cyclopentanone de formule (I) ci-dessus.

On entend par "cheveux sensibilisés" des cheveux ayant subi un traitement de permanente, de coloration ou de décoloration.

L'invention vise aussi une composition cosmétique colorée ou non colorée, stabilisée à la lumière, comprenant une quantité efficace d'au moins un dérivé de 5-benzylidène 3-oxa cyclopentanone de formule (I) ci-dessus.

L'invention a également pour objet les nouveaux dérivés de 5-benzylidène 3-oxa cyclopentanone de formule générale (I') suivante :

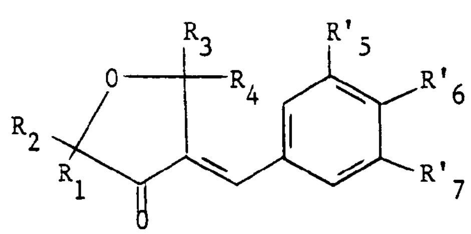

(I')

dans laquelle :

$R_1$, $R_2$, $R_3$ et $R_4$ ont les mêmes significations que dans la formule (I); $R'_5$ et $R'_7$, identiques ou différents, représentent un atome d'hydrogène, un reste alkyle linéaire ou ramifié en $C_1$-$C_8$, un reste alcényle linéaire ou ramifié en $C_2$-$C_8$, un reste alcoxy linéaire ou ramifié en $C_1$-$C_{12}$, un reste alcényloxy linéaire ou ramifié en $C_2$-$C_8$, un reste acyloxy linéaire ou ramifié en $C_2$-$C_{12}$, un reste benzyloxy non substitué ou substitué par des atomes

d'halogène, des groupes alkyle ou alcoxy en $C_1$-$C_4$, ou un reste $COOR_{10}$ où $R_{10}$ désigne un atome d'hydrogène, un radical alkyle linéaire ou ramifié en $C_1$-$C_8$, un métal alcalin, alcalino-terreux ou un radical dérivé d'amine;

$R'_6$ représente un reste alkyle linéaire ou ramifié en $C_2$-$C_8$, un reste alcényle linéaire ou ramifié en $C_2$-$C_8$, un reste alcoxy linéaire ou ramifié en $C_2$-$C_{12}$, un reste alcényloxy linéaire ou ramifié en $C_2$-$C_8$, un reste acyloxy linéaire ou ramifié en $C_2$-$C_{12}$, un reste benzyloxy non substitué ou substitué par des atomes d'halogène, des groupes alkyle ou alcoxy en $C_1$-$C_4$, un reste $-COOR_{10}$ où $R_{10}$ désigne un radical alkyle linéaire ou ramifié en $C_1$-$C_8$, un atome d'hydrogène, de métal alcalin ou alcalinoterreux ou un radical dérivé d'amine, ou encore un radical de formule :

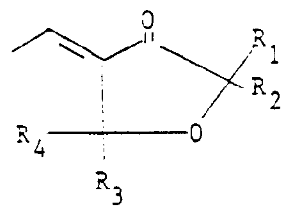

où $R_1$, $R_2$, $R_3$ et $R_4$ significations mentionnées ci-dessus pour la formule (I).

On préfère plus particulièrement les composés de formule (I') présentant au moins l'une des caractéristiques suivantes :

- $R_1$, $R_2$, $R_3$ et $R_4$ sont des radicaux méthyle,
- $R'_5$ et $R'_7$ représentent un atome d'hydrogène, un radical benzyloxy ou méthoxy,
- $R'_6$ représente un radical n-butoxy, tert.-butoxy. hexyloxy, octyloxy, allyloxy, benzyloxy, méthoxycarbonyle, (2,2,4,4-tétraméthyl-3-oxa-5-oxo-1-cyclopentylidène)méthyle, carboxy.

Parmi les composés nouveaux de formule (I') on peut citer les composés préférés suivants :

- la 5-(4'-hexyloxy benzylidène) 3-oxa 2,2,4,4-tétraméthyl cyclopentanone,
- la 5-(4'-allyloxy benzylidène) 3-oxa 2,2,4,4-tétraméthyl cyclopentanone,
- la 5-(4'-octyloxy benzylidène) 3-oxa 2,2,4,4-tétraméthyl cylopentanone,
- la 5-(3',4',5'-tribenzyloxy benzylidène) 3-oxa 2,2,4,4- tétraméthyl cyclopentanone,
- la 5-(4'-tert.-butoxy benzylidène) 3-oxa 2,2,4,4- tétraméthyl cyclopentanone,
- la 5-(4'-méthoxycarbonyl benzylidène) 3-oxa 2,2,4,4-tétraméthyl cyclopentanone,
- la 5-(4'-n-butoxy 5'-méthoxy benzylidène) 3-oxa 2,2,4,4-tétraméthyl cyclopentanone,
- le 1,4-bis-[(2',2',4',4'-tétraméthyl-3'-oxa-5'-oxo-1'-cyclopentylidène)méthyl] benzène,
- l'acide 4-[(2',2',4',4'-tétraméthyl-3'-oxa-5'-oxo-1'-cyclopentylidène)méthyl] benzoïque.

Les composés de formule (I) ou (I') sont obtenus par condensation d'un aldéhyde aromatique de formule (II) ou (II') sur une 3-oxa cyclopentanone de formule (III) :

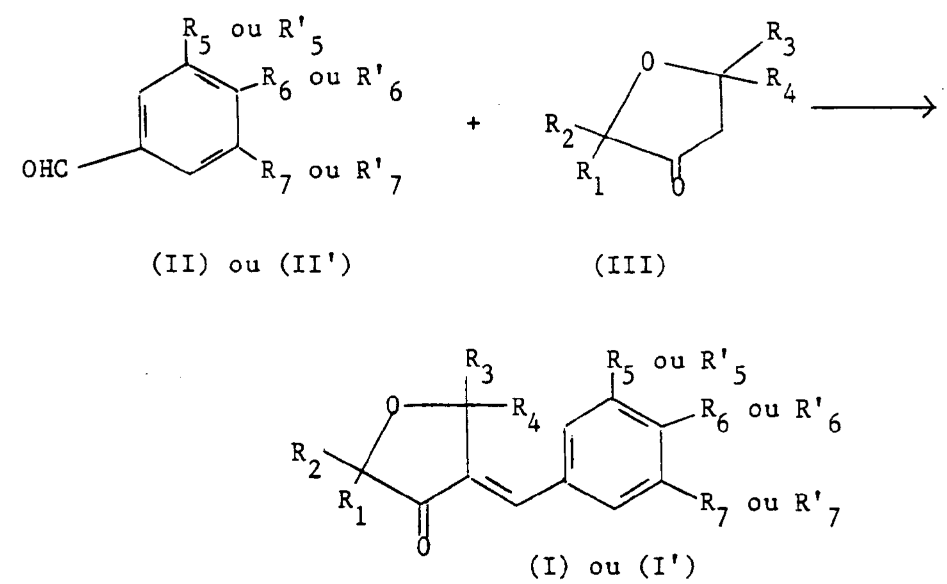

(II) ou (II')

(III)

(I) ou (I')

Dans les composés de formule (II), (II') et (III), les substituants $R_1$ à $R_7$ ou $R_1$ à $R'_7$ ont les significations

mentionnées ci-dessus pour les composés de formule (I) et (I′).

Les aldéhydes de formule (II) ou (II′) sont des composés connus. Les 3-oxa cyclopentanones de formule (III) peuvent être préparées selon les méthodes décrites par C. SANDRIS et G. OURISSON, Bull. Soc. Chim. Fr. (1956), p.958 et par I.K KOROBITSYNA et Coll., Zhur. Obschei Khim. Vol. 25, p. 734-738 et Vol. 27, p. 1792-1795.

La condensation de l'aldéhyde (II) ou (II′) sur l'oxa-3 cyclopentanone (III) peut être réalisée selon l'un des deux procédés suivants :

PREMIER PROCEDE

La condensation est effectuée en présence d'un alcoolate de métal alcalin tel que le méthylate de sodium ou le tert.-butylate de potassium, dans un solvant tel que le toluène ou le 1,2-diméthoxy éthane à une température comprise entre -78°C et le point d'ébullition du solvant. La condensation peut également être effectuée en présence d'une base minérale telle qu'un amidure ou un hydrure de métal alcalin, dans un solvant comme le 1,2-diméthoxy éthane, ou un hydroxyde de métal alcalin dans un alcool, à une température comprise entre 0°C et le point d'ébullition du mélange réactionnel.

DEUXIEME PROCEDE

La condensation de l'aldéhyde (II) ou (II′) sur la 3-oxa cyclopentanone (III) est effectuée en présence d'un borane de formule (IV) suivante :

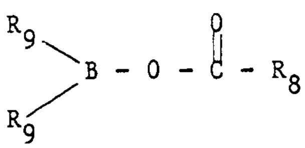

(IV)

dans laquelle $R_8$ représente un reste alkyle en $C_1$-$C_6$ et $R_9$ représente un reste alkyle en $C_1$-$C_4$. Ce composé est obtenu selon le mode opératoire décrit par L.H. TOPORCER et al., J. Am. Chem. Soc. Vol. 87, p. 1236, (1965). Son isolement et sa purification ne sont pas nécessaires pour réaliser la condensation de l'aldéhyde (II) ou (II′) sur la 3-oxa cyclopentanone (III).

La réaction de condensation est effectuée à une température de 150°C environ, sans solvant.

Les composés (I) ou (I′) dans lesquels l'un des radicaux $R_8$, $R_6$ ou $R_7$ ou $R'_5$ à $R'_7$ désigne le reste -$COOR_{10}$ où $R_{10}$ représente un atome d'hydrogène, sont obtenu par saponification des esters correspondants.

La présente invention a donc également pour objet le procédé de préparation des composés nouveaux de formule (I′).

La composition cosmétique de l'invention peut être utilisée comme composition protectrice de l'épiderme humain ou des cheveux ou comme composition antisolaire.

Lorsqu'elle est utilisée comme composition destinée à protéger l'épiderme humain contre les rayons ultraviolets, la composition cosmétique selon l'invention peut se présenter sous les formes les plus diverses habituellement utilisées pour ce type de composition. Elle peut notamment se présenter sous forme de lotion, d'émulsion telle qu'une crème ou un lait, d'huile, de gel, de bâtonnet solide ou être conditionnée en aérosol.

Elle peut contenir les adjuvants cosmétiques habituellement utilisés dans ce type de composition tels que des épaississants, des adoucissants, des humectants, des tensio-actifs, des conservateurs, des anti-mousses, des parfums, des huiles, des cires, de la lanoline, des propulseurs, des colorants et/ou pigments ayant pour fonction de colorer la composition elle-même ou la peau ou tout autre ingrédient habituellement utilisé en cosmétique.

Le composé de formule (I) est présent dans des proportions comprises entre 0,25 et 3% en poids par rapport au poids total de la composition cosmétique protectrice de l'épiderme humain.

Comme solvant de solubilisation, on peut utiliser une huile, une cire et de façon générale tout corps gras, un monoalcool ou un polyol inférieur ou leur mélange. Les monoalcools ou polyols plus particulièrement préférés sont l'éthanol, l'isopropanol, le propylène glycol, la glycérine et le sorbitol.

Une forme de réalisation de l'invention est une émulsion sous forme de crème ou de lait protecteurs comprenant en plus du composé de formule (I), des alcools gras, des esters d'acides gras et notamment des triglycérides d'acides gras, des acides gras, de la lanoline, des huiles ou cires naturelles ou synthétiques et des émulsionnants, en présence d'eau.

Une autre forme de réalisation est constituée par des lotions huileuses à base d'huiles et cires naturelles

ou synthétiques, de lanoline, et d'esters d'acides gras, notamment de triglycérides d'acides gras, ou par des lotions oléoalcooliques à base d'un alcool inférieur tel que l'éthanol ou d'un glycol tel que le propylèneglycol et/ou d'un polyol tel que la glycérine et d'huiles, de cires et d'esters d'acides gras tels que les triglycérides d'acides gras.

La composition cosmétique de l'invention peut également être un gel alcoolique ou hydroalcoolique comprenant un ou plusieurs alcools ou polyols inférieurs tels que l'éthanol, le propylèneglycol ou la glycérine et un épaississant tel que la silice, éventuellement en présence d'eau. Les gels oléoalcooliques contiennent en outre une huile ou une cire naturelle ou synthétique.

Les bâtonnets solides sont constitués de cires et d'huiles naturelles ou synthétiques, d'alcools gras, d'esters d'acides gras, de lanoline et autres corps gras.

La présente invention vise également les compositions cosmétiques antisolaires contenant au moins un composé de formule (I) qui peut être associé à d'autres filtres UV-B et/ou UV-A.

Dans ce cas, la quantité totale de filtres présents dans la composition antisolaire est comprise entre 0,5 et 15% en poids par rapport au poids total de la composition. Ces compositions cosmétiques anti-solaires se présentent sous les mêmes formes que les compositions destinées à protéger l'épiderme humain décrites ci-dessus.

Lorsque la composition cosmétique selon l'invention est destinée à protéger des rayons UV les cheveux naturels ou sensibilisés, cette composition peut se présenter sous forme de shampooing, lotion, gel ou émulsion à rincer, à appliquer avant ou après shampooing, avant ou après coloration ou décoloration, avant ou après permanente, de lotion ou gel coiffants ou traitants, de lotion ou gel pour le brushing ou la mise en plis, de laque pour cheveux, de composition de permanente, de coloration ou décoloration des cheveux. Elle contient 0,25 à 4% en poids de composé de formule (I).

La présente invention vise également des compositions cosmétiques colorées ou non, contenant au moins un composé de formule (I) à titre d'agent de protection contre les rayons UV.

Ces compositions peuvent être constituées par des produits pour cheveux tels que les laques pour cheveux, les lotions de mise en plis éventuellement traitantes ou démêlantes, les shampooings, les shampooings colorants, les compositions tinctoriales pour cheveux ou par des produits de maquillage tels que les vernis à ongles, les crèmes de traitement pour l'épiderme, les fonds de teint, les bâtons de rouge à lèvres, ainsi que par toute composition cosmétique pouvant présenter du fait de ses constituants des problèmes de stabilité à la lumière au cours du stockage. Elles contiennent 0,25 à 3% en poids de composé de formule (I).

L'invention est illustrée par les exemples non limitatifs ci-après.

## EXEMPLES DE PREPARATION

### EXEMPLE 1

5-(4'-hexyloxy benzylidène)3-oxa 2,2,4,4-tétraméthyl cyclopentanone

Préparation d'un composé de formule générale (I') dans laquelle $R_1 = R_2 = R_3 = R_4 = CH_3$, $R'_5 = R'_7 = H$ et $R'_6 = -O-C_6H_{13}$ :

On dissout 104 g de 2,2,4,4-tétraméthyl 3-oxa cyclopentanone (0,72 mole) dans 100 $cm^3$ de 1,2-diméthoxy éthane. On refroidit vers 5°C puis on ajoute 39 g (0,72 mole) de méthylate de sodium sous agitation. On introduit goutte à goutte, à une température comprise entre 5° et 10°C, une solution de 134 g (0,65 mole) de 4-hexyloxy benzaldéhyde dans 100 $cm^3$ de 1,2-diméthoxy éthane. On agite pendant 3 heures à une température voisine de 0°C puis on verse le mélange réactionnel dans de l'eau glacée. Le précipité est filtré, lavé abondamment à l'eau et séché sous pression réduite. Après recristallisation dans l'heptane, le produit attendu se présente sous forme de cristaux jaune pâle possédant les caractéristiques suivantes :

- Point de fusion : 63°C
- Spectre UV (chloroforme) :

$\lambda_{max}$ : 335 nm

$\varepsilon$ : 23000

- Analyse élémentaire : $C_{21}H_{30}O_3$

| | C% | H% | O% |
|---|---|---|---|
| Calculé | 76,33 | 9,15 | 14,52 |
| Trouvé | 76,19 | 9,17 | 14,80 |

EXEMPLE 2

5-benzylidène 3-oxa 2,2,4,4-tétraméthyl cyclopentanone

Préparation d'un composé de formule générale (I) dans laquelle $R_1$= $R_2$= $R_3$= $R_4$= $CH_3$et $R_5$= $R_6$= $R_7$= H :

Ce composé est obtenu selon le mode opératoire décrit à l'exemple 1 dans lequel le 4-hexyloxy benzaldéhyde est remplacé par le benzaldéhyde. Le produit obtenu possède les caractéristiques suivantes :
- Point de fusion : 78°C
- Spectre UV (dichlorométhane) :

$\lambda_{max}$ : 299 nm
ε : 14600

- Analyse élémentaire : $C_{15}H_{18}O_2$

| | C% | H% | O% |
|---|---|---|---|
| Calculé | 78,23 | 7,88 | 13,89 |
| Trouvé | 77,66 | 7,96 | 14,46 |

EXEMPLE 3

5-(4'-allyloxy benzylidène) 3-oxa 2,2,4,4-tétraméthyl cyclopentanone

Préparation d'un composé de formule générale (I') dans laquelle $R_1$= $R_2$= $R_3$= $R_4$= $CH_3$,$R'_5$= $R'_7$= H et $R'_6$= $-O-CH_2-CH=CH_2$ :

Ce composé est obtenu selon le mode opératoire décrit à l'exemple 1 dans lequel le 4-hexyloxy benzaldéhyde est remplacé par le 4-allyloxy benzaldéhyde. Le produit obtenu possède les caractéristiques suivantes :
- Point de fusion : 82°C
- Spectre UV (dichlorométhane) :

$\lambda_{max}$ : 330 nm
ε : 23450

- Analyse élémentaire : $C_{18}H_{22}O_3$

| | C% | H% | O% |
|---|---|---|---|
| Calculé | 75,50 | 7,74 | 16,76 |
| Trouvé | 75,03 | 7,75 | 17,41 |

EXEMPLE 4

5-(4'-octyloxy benzylidène) 3-oxa 2,2,4,4-tétraméthyl cyclopentanone

Préparation d'un composé de formule générale (I') dans laquelle $R_1$= $R_2$= $R_3$= $R_4$= $CH_3$,$R'_5$= $R'_7$= H et $R'_6$= $-O-C_8H_{17}$

Ce composé est obtenu selon le mode opératoire décrit à l'exemple 1 dans lequel le 4-hexyloxy benzaldéhyde est remplacé par le 4-octyloxy benzaldéhyde. Le produit obtenu possède les caractéristiques suivantes :

- Point de fusion : 50°C
- Spectre UV (dichlorométhane) :

$\lambda_{max}$ : 332 nm
ε : 23350

- Analyse élémentaire : $C_{23}H_{34}O_3$

| | C% | H% | O% |
|---|---|---|---|
| Calculé | 77,09 | 9,50 | 13,41 |
| Trouvé | 77,11 | 9,62 | 13,41 |

EXEMPLE 5

5-(3',4',5'-tribenzyloxy benzylidène) 3-oxa 2,2,4,4-tétraméthyl cyclopentanone

Préparation d'un composé de formule générale (I') dans laquelle $R_1$= $R_2$= $R_3$= $R_4$= $CH_3$et $R'_5$= $R'_6$= $R'_7$= $-O-CH_2-C_6H_5$

Ce composé est obtenu selon le mode opératoire décrit à l'exemple 1 dans lequel le 4-hexyloxy benzaldéhyde est remplacé par le 3,4,5-tribenzyloxy benzaldéhyde. Le produit obtenu possède les caractéristiques suivantes :

- Point de fusion : 73-75°C
- Spectre UV (dichlorométhane) :

$\lambda_{max}$ : 340 nm
ε : 11410

- Analyse élémentaire : $C_{36}H_{36}O_5$

| | C% | H% | O% |
|---|---|---|---|
| Calculé | 78,81 | 6,61 | 14,58 |
| Trouvé | 78,78 | 6,61 | 14,64 |

EXEMPLE 6

5-(4'-tert.-butoxy benzylidène) 3-oxa 2,2,4,4-tétraméthyl cyclopentanone

Préparation d'un composé de formule générale (I') dans laquelle $R_1$= $R_2$= $R_3$= $R_4$= $CH_3$,$R'_5$= $R'_7$= H et $R'_6$= -O-tert.-butyle :

Ce composé est obtenu selon le mode opératoire décrit à l'exemple 1 dans lequel le 4-hexyloxy benzaldéhyde est remplacé par le 4-tert.-butoxy benzaldéhyde. Le produit obtenu possède les caractéristiques suivantes :

- Point de fusion : 72°C
- Spectre UV (dichlorométhane) :

$\lambda_{max}$ : 326 nm
ε : 34275

- Analyse élémentaire : $C_{19}H_{26}O_3$

| | C% | H% | O% |
|---|---|---|---|
| Calculé | 75,46 | 8,67 | 15,87 |
| Trouvé | 74,83 | 8,72 | 16,49 |

EXEMPLE 7

5-(4'-méthoxycarbonyl benzylidène) 3-oxa 2,2,4,4-tétraméthyl cyclopentanone

Préparation d'un composé de formule générale (I') dans laquelle $R_1= R_2= R_3= R_4= CH_3, R'_5= R'_7=$ H et $R'_6= -CO_2CH_3$ :

Ce composé est obtenu selon le mode opératoire décrit à l'exemple 1 dans lequel le 4-hexyloxy benzaldéhyde est remplacé par le 4-formyl benzoate de méthyle. Le produit obtenu possède les caractéristiques suivantes :

- Point de fusion : 108-110°C
- Spectre UV (dichlorométhane) :

$\lambda_{max}$ : 300 nm
ε : 16200

- Analyse élémentaire : $C_{17}H_{20}O_4$

| | C% | H% | O% |
|---|---|---|---|
| Calculé | 70,81 | 6,99 | 22,19 |
| Trouvé | 70,67 | 7,06 | 22,28 |

EXEMPLE 8

5-(4'-n-butoxy 5'-méthoxy benzylidène) 3-oxa 2,2,4,4-tétraméthyl cyclopentanone

Préparation d'un composé de formule générale (I') dans laquelle $R_1= R_2= R_3= R_4= CH_3, R'_5=$ H, $R'_6= -O-C_4H_9$ et $R'_7= -OCH_3$ :

Ce composé est obtenu selon le mode opératoire décrit à l'exemple 1 dans lequel le 4-hexyloxy benzaldéhyde est remplacé par le 4-butoxy 3-méthoxy benzaldéhyde. Le produit obtenu possède les caractéristiques suivantes :

- Point de fusion : 102°C
- Spectre UV (chloroforme) :

$\lambda_{max}$ : 350 nm
ε : 20000

- Analyse élémentaire : $C_{20}H_{28}O_4$

| | C% | H% | O% |
|---|---|---|---|
| Calculé | 72,26 | 8,49 | 19,25 |
| Trouvé | 72,42 | 8,42 | 19,20 |

EXEMPLE 9

1,4-Bis-[(2′,2′,4′,4′-tétraméthyl-3′-oxa-5′-oxo-1′-cyclopentylidène)méthyl] benzène

Préparation d'un composé de formule générale (I′) dans laquelle $R_1$= $R_2$= $R_3$= $R_4$= $CH_3$,$R'_5$= $R'_7$= H et $R'_6$ représente un radical de formule

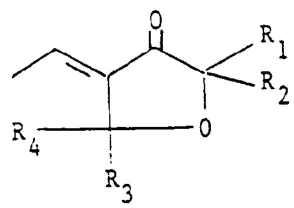

Ce composé est obtenu selon le mode opératoire décrit à l'exemple 1 dans lequel le 4-hexyloxy benzaldéhyde est remplacé par l'aldéhyde téréphtalique. Le produit obtenu possède les caractéristiques suivantes :

- Point de fusion : 262°C
- Spectre UV (chloroforme) :

$\lambda_{max}$ : 330 nm

ε : 25000

- Analyse élémentaire : $C_{24}H_{30}O_4$, 0,25 $H_2O$

| | C% | H% | O% |
|---|---|---|---|
| Calculé | 74,51 | 7,89 | 17,59 |
| Trouvé | 74,46 | 7,99 | 17,54 |

EXEMPLE 10

Acide 4-[(2′,2′,4′,4′-tétraméthyl-3′-oxa-5′-oxo-cyclopentylidène)méthyl] benzoïque

Préparation d'un composé de formule générale (I′) dans laquelle $R_1$= $R_2$= $R_3$= $R_4$= $CH_3$,$R'_5$= $R'_7$= H et $R'_6$= $-CO_2H$ :

On chauffe pendant 30 minutes au reflux 5 g de composé obtenu à l'exemple 7 dans 100 $cm^3$ de mélange éthanol-eau 50/50 contenant 1 g de potasse. Le mélange réactionnel est versé dans l'eau glacée. Après acidification par addition d'acide chlorhydrique 1N, filtration du précipité, séchage et recristallisation dans l'acétate d'éthyle, on obtient le produit attendu sous forme de cristaux jaune-pâle possédant les caractéristiques suivantes :

- Point de fusion > 250°C
- Spectre UV (dichlorométhane) :

$\lambda_{max}$ : 298 nm

ε : 15710

- Analyse élémentaire : $C_{16}H_{18}O_4$

| | C% | H% | O% |
|---|---|---|---|
| Calculé | 70,06 | 6,61 | 23,33 |
| Trouvé | 69,96 | 6,57 | 23,48 |

EXEMPLES D'APPLICATION

Exemple A

Crème de jour protectrice :

| | | |
|---|---|---|
| - Composé de l'exemple 1 | 1 | g |
| - Alcools gras polyoxyéthylénés * | 7 | g |
| - Triglycérides d'acides gras $C_8$-$C_{12}$ | 30 | g |
| - Monostéarate de glycérol | 2 | g |
| - Huile de silicone | 1,5 | g |
| - Alcool cétylique | 1,5 | g |
| - Conservateurs | 0,3 | g |
| - Parfum | 0,6 | g |
| - Eau déminéralisée qsp | 100 | g |

* Alcools gras polyoxyéthylénés : mélange d'alcool cétylstéarylique et d'alcool cétylstéarylique oxyéthyléné à 33 moles d'oxyde d'éthylène.

Pour la préparation de cette crème, on chauffe les corps gras vers 80-85°C; on ajoute le filtre de formule (I). D'autre part, on chauffe l'eau à 80-85°C et sous vive agitation, on ajoute la phase grasse à la phase aqueuse; on maintient l'agitation pendant 10 à 15 minutes, puis on laisse refroidir sous agitation modérée et vers 40°C, on ajoute parfum et conservateurs.

Exemple B

Lait protecteur :

| | | |
|---|---|---|
| - Composé de l'exemple 4 | 1 | g |
| - p-diméthylamino-benzoate d'octyle | 0,5 | g |
| - Alcool cétylstéarylique | 2 | g |
| - Alcool cétylique | 2 | g |
| - Triglycérides d'acides gras ($C_8$ à $C_{12}$) | 20 | g |
| - Lanoline | 4 | g |
| - Acide stéarique | 0,5 | g |
| - Conservateurs | 0,3 | g |
| - Carbopol 934 (acide polyacrylique réticulé vendu par la Société GOODRICH CHEMICAL) | 0,15 | g |
| - Triéthanolamine | 0,2 | g |
| - Parfum | 0,4 | g |
| - Eau déminéralisée qsp | 100 | g |

On chauffe les corps gras vers 80-85°C; on ajoute les filtres. D'autre part, on chauffe l'eau à 80-85°C et sous vive agitation, on ajoute la phase grasse à la phase aqueuse (contenant les composés hydrosolubles); on maintient l'agitation pendant 10 à 15 minutes, puis on laisse refroidir sous agitation modérée et vers 40°C, on ajoute parfum et conservateurs.

Exemple C

Crème solaire :

| | | |
|---|---|---|
| - Composé de l'exemple 1 | 4 | g |
| - Méthylsulfate de 4[(2-oxo-3-bornylidène)-méthyl] phényltriméthylammonium | 3,5 | g |
| - Alcools gras polyoxyéthylénés * | 7 | g |
| - Triglycérides d'acides gras ($C_8$ à $C_{12}$) | 30 | g |
| - Monostéarate de glycérol | 2 | g |
| - Huile de silicone | 1,5 | g |
| - Alcool cétylique | 1,5 | g |
| - Conservateurs, parfum qs | | |
| - Eau déminéralisée | 100 | g |

* Alcools gras polyoxyéthylénés : mélange d'alcool cétylstéarylique et d'alcool cétylstéarylique oxyéthyléné à 33 moles d'oxyde d'éthylène.

La préparation de cette crème est similaire à celle de l'exemple A, le méthylsufate de 4[(2-oxo-3-bornylidène)méthyl]phényltriméthylammonium étant dissous dans l'eau.

On peut remplacer le composé de l'exemple 1 par le composé de l'exemple 6.

Exemple D

Gel solaire hydro-alcoolique :

| | | | |
|---|---|---|---|
| - Sel de diéthanolamine du composé de l'exemple 10 | | 1 | g |
| - Sel de diéthanolamine de l'acide p-méthoxycinnamique | | 2,5 | g |
| - Carbopol 934 | | 0,7 | g |
| - Triéthanolamine | | 0,35 | g |
| - Propylène glycol | | 25 | g |
| - Ethanol à 96° | | 25 | g |
| - Conservateur | | 0,3 | g |
| - Parfum | | 0,4 | g |
| - Eau déminéralisée | qsp | 100 | g |

On disperse le Carbopol sous vive agitation dans 30 g d'eau, puis on ajoute la triéthanolamine et ensuite les solvants et le restant d'eau dans lesquels on a préalablement dissous les filtres.

Exemple E

Lotion oléo-alcoolique :

| | | | |
|---|---|---|---|
| - Composé de l'exemple 8 | | 1,5 | g |
| - p-méthoxycinnamate de 2-éthylhexyle | | 2 | g |
| - Parfum | | 0,5 | g |
| - Ethanol à 96° | | 47,5 | g |
| - Triglycérides d'acides gras ($C_8$ à $C_{12}$) | qsp | 100 | g |

On chauffe le mélange des différents constituants vers 40-45°C pour homogénéiser et obtenir une lotion limpide. On laisse refroidir, puis on ajoute le parfum.

Exemple F

Huile solaire :

On mélange les ingrédients suivants en chauffant éventuellement à 40-45°C pour homogénéiser :

| | | | |
|---|---|---|---|
| - Composé de l'exemple 1 | | 3 | g |
| - p-diméthylamino-benzoate d'octyle | | 3 | g |
| - Beurre de cacao | | 2,5 | g |
| - Antioxydants | | 0,05 | g |
| - Parfum | | 0,5 | g |
| - Triglycérides d'acides gras ($C_8$ à $C_{12}$) | qsp | 100 | g |

On peut remplacer le composé de l'exemple 1 par 3 g de composé de l'exemple 4.

Exemple G

Crème solaire :

| | | | |
|---|---|---|---|
| - Composé de l'exemple 1 | | 2,5 | g |
| - Benzylidène camphre | | 4 | g |
| - Triglycérides d'acides gras ($C_8$ à $C_{12}$) | | 31 | g |
| - Monostéarate de glycérol | | 6 | g |
| - Acide stéarique | | 2 | g |
| - Alcool cétylique | | 1,2 | g |
| - Lanoline | | 4 | g |
| - Conservateurs | | 0,3 | g |
| - Propanediol | | 2 | g |
| - Triéthanolamine | | 0,5 | g |
| - Parfum | | 0,5 | g |
| - Eau déminéralisée | qsp | 100 | g |

L'émulsion est préparée de la même façon que dans l'exemple A.

Exemple H

Lait solaire :

| | | |
|---|---|---|
| - Composé de l'exemple 1 | 1 | g |
| - Composé de l'exemple 8 | 3 | g |
| - Alcool cétylstéarylique | 2 | g |
| - Alcool cétylique | 2 | g |
| - Triglycérides d'acides gras ($C_8$ à $C_{12}$) | 20 | g |
| - Lanoline | 4 | g |
| - Acide stéarique | 0,5 | g |
| - Conservateurs | 0,3 | g |
| - Carbopol 934 (acide polyacrylique réticulé vendu par la Société GOODRICH CHEMICAL) | 0,15 | g |
| - Triéthanolamine | 0,2 | g |
| - Parfum | 0,4 | g |
| - Eau déminéralisée qsp | 100 | g |

L'émulsion est préparée de la même façon que dans l'exemple A.

## Revendications

**1.** Composition cosmétique caractérisée par le fait qu'elle comprend, dans un support cosmétiquement acceptable, à titre de composé filtrant le rayonnement ultraviolet de longueurs d'onde comprises entre 280 et 380 nm, une quantité efficace d'au moins un dérivé de 5-benzylidène 3-oxa cyclopentanone de formule générale :

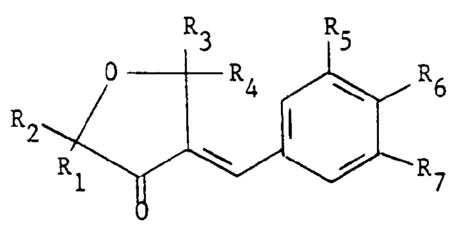
(I)

dans laquelle :

$R_1$, $R_2$, $R_3$ et $R_4$, identiques ou différents, représentent un reste alkyle linéaire ou ramifié en $C_1$-$C_8$, un reste aralkyle non substitué ou substitué par des atomes d'halogène ou par des restes alkyle ou alcoxy en $C_1$-$C_4$, ou un reste aryle non substitué ou substitué par des atomes d'halogène, des restes alkyle ou alcoxy en $C_1$-$C_4$, ou bien $R_1$ et $R_2$ et/ou $R_3$ et $R_4$ forment avec l'atome de carbone auquel ils sont rattachés, un cycle saturé contenant 5 ou 6 atomes de carbone;

$R_5$, $R_5$ et $R_7$ identiques ou différents, représentent un atome d'hydrogène, un reste alkyle linéaire ou ramifié en $C_1$-$C_8$, un reste alcényle linéaire ou ramifié en $C_2$-$C_8$, un reste alcoxy linéaire ou ramifié en $C_1$-$C_{12}$, un reste alcényloxy linéaire ou ramifié en $C_2$-$C_8$, un reste acyloxy linéaire ou ramifié en $C_2$-$C_{12}$, un reste benzyloxy non substitué ou substitué par des atomes d'halogène ou par des restes alkyle ou alcoxy en $C_1$-$C_4$, ou un reste -$COOR_{10}$ où $R_{10}$ désigne un reste alkyle linéaire ou ramifié en $C_1$-$C_8$, un atome d'hydrogène, de métal alcalin ou alcalino-terreux ou un radical dérivé d'amine, l'un des substituants $R_5$, $R_6$ et $R_7$ pouvant également représenter un radical de formule :

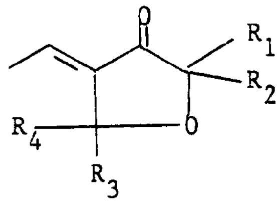

dans laquelle $R_1$, $R_2$, $R_3$ et $R_4$ ont les significations mentionnées ci-dessus.

2. Composition cosmétique selon la revendication 1, caractérisée par le fait qu'elle comprend à titre de composé de formule (I) au moins l'un des composés suivants : la 5-benzylidène 3-oxa 2,2,4,4-tétraméthyl cyclopentanone, la 5-(4'-hexyloxy benzylidène) 3-oxa 2,2,4,4-tétraméthylcyclopentanone, la 5-(4'-allyloxy benzylidène) 3-oxa 2,2,4,4-tétraméthyl cyclopentanone, la 5-(4'-octyloxy benzylidène) 3-oxa 2,2,4,4-tétraméthyl cylopentanone, la 5-(3',4', 5'-tribenzyloxy benzylidène) 3-oxa 2,2,4,4-tétraméthyl cyclopentanone, la 5-(4'-tert.-butoxy benzylidène) 3-oxa 2,2,4,4-tétraméthyl cyclopentanone, la 5-(4'-méthoxycarbonyl benzylidène) 3-oxa 2,2,4,4- tétraméthyl cyclopentanone, la 5-(4'-n-butoxy 5'-méthoxy benzylidène) 3-oxa 2,2,4,4-tétraméthyl cyclopentanone, le 1,4-bis-[(2', 2',4',4'-tétraméthyl-3'-oxa-5'-oxo-1'-cyclopentylidène)méthyl] benzène, l'acide 4-[(2',2',4',4'-tétraméthyl-3'-oxa-5'oxo-1'-cyclopentylidène)méthyl] benzoïque.

3. Composition cosmétique selon la revendication 1 ou 2, caractérisée par le fait qu'elle se présente sous forme de lotion, émulsion, huile, gel, bâtonnet solide ou aérosol.

4. Composition cosmétique selon l'une quelconque des revendications 1 à 3, caractérisée par le fait qu'elle contient en outre des adjuvants cosmétiques choisis parmi les épaississants, adoucissants, humectants, tensio-actifs, conservateurs, anti-mousses, parfums, huiles, cires, lanoline, mono-alcools et polyols inférieurs, propulseurs, colorants et pigments.

5. Composition cosmétique selon l'une quelconque des revendications 1 à 4, se présentant sous forme de composition protectrice de l'épiderme humain, caractérisée par le fait qu'elle contient 0,25 à 3% en poids d'au moins un composé de formule (I).

6. Composition cosmétique selon l'une quelconque des revendications 1 à 4, se présentant sous forme de composition antisolaire, caractérisée par le fait qu'elle contient 0,5 à 15% en poids d'au moins un composé de formule (I).

7. Composition cosmétique selon la revendication 6, caractérisée par le fait qu'elle contient en outre d'autres filtres solaires filtrant les rayons UV-B ou UV-A.

8. Composition cosmétique selon la revendication 1 ou 2, destinée à être appliquée sur les cheveux, caractérisée par le fait qu'elle se présente sous forme de shampooing, lotion, gel ou émulsion à rincer, lotion ou gel coiffants ou traitants, lotion ou gel pour le brushing ou la mise en plis, laque pour cheveux, composition de permanente, de décoloration ou de coloration et comprend 0,25 à 4% en poids d'au moins un composé de formule (I).

9. Composition cosmétique selon la revendication 1 ou 2, se présentant sous forme d'une composition cosmétique colorée ou non, stabilisée à la lumière, caractérisée par le fait qu'elle est constituée par une laque pour cheveux, une lotion pour mise en plis, un shampooing, un shampooing colorant, une composition tinctoriale pour cheveux ou un produit de maquillage tel qu'un vernis à ongles, un bâton de rouge à lèvres, une crème de traitement pour l'épiderme ou un fond de teint, et contient 0,25 à 3% en poids d'au moins un composé de formule (I).

10. Procédé de traitement cosmétique de la peau et des cheveux naturels ou sensibilisés en vue de les protéger du rayonnement ultraviolet de longueurs d'onde comprises entre 280 et 380 nm, caractérisé par le fait qu'il consiste à appliquer sur la peau ou les cheveux une quantité efficace d'une composition cosmétique contenant au moins un composé de formule (I) selon la revendication 1 ou 2.

11. Procédé de protection d'une composition cosmétique contre les rayons ultraviolets de longueurs d'onde

comprises entre 280 et 380 nm, caractérisé par le fait qu'il consiste à incorporer à cette composition une quantité efficace d'au moins un composé de formule (I) selon la revendication 1 ou 2.

**12.** Dérivé de 5-benzylidène 3-oxa cyclopentanone de formule générale :

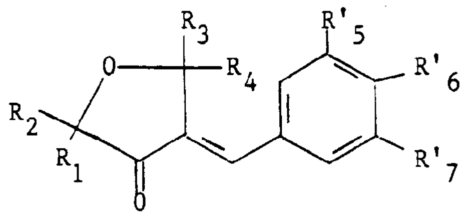

(I')

dans laquelle :

$R_1$, $R_2$, $R_3$ et $R_4$ identiques ou différents représentent un reste alkyle linéaire ou ramifié en $C_1$-$C_8$, un reste aralkyle non substitué ou substitué par des atomes d'halogène, des restes alkyle ou alcoxy en $C_1$-$C_4$, ou un reste aryle non substitué ou substitué par des atomes d'halogène, des restes alkyle ou alcoxy en $C_1$-$C_4$, ou bien $R_1$ et $R_2$ et/ou $R_3$ et $R_4$ forment avec l'atome de carbone auquel ils sont rattachés un cycle saturé comportant 5 ou 6 atomes de carbone;

$R'_5$ et $R'_7$, identiques ou différents, représentent un atome d'hydrogène, un reste alkyle linéaire ou ramifié en $C_1$-$C_8$, un reste alcényle linéaire ou ramifié en $C_2$-$C_8$, un reste alcoxy linéaire ou ramifié en $C_1$-$C_{12}$, un reste alcényloxy linéaire ou ramifié en $C_2$-$C_8$, un reste acyloxy linéaire ou ramifié en $C_2$-$C_{12}$, un reste benzyloxy non substitué ou substitué par des atomes d'halogène, des groupes alkyle ou alcoxy en $C_1$-$C_4$, ou un reste -$COOR_{10}$ où $R_{10}$ est un reste alkyle linéaire ou ramifié en $C_1$-$C_8$ ou désigne un atome d'hydrogène, de métal alcalin, alcalino terreux ou un radical dérivé d'amine;

$R'_6$ représente un reste alkyle linéaire ou ramifié en $C_2$-$C_8$, un reste alcényle linéaire ou ramifié en $C_2$-$C_8$, un reste alcoxy linéaire ou ramifié en $C_2$-$C_{12}$, un reste alcényloxy linéaire ou ramifié en $C_2$-$C_8$, un reste acyloxy linéaire ou ramifié en $C_2$-$C_{12}$, un reste benzyloxy non substitué ou substitué par des atomes d'halogène, ou des radicaux alkyle ou alcoxy en $C_1$-$C_4$, un reste -$COOR_{10}$ où $R_{10}$ désigne un radical alkyle en $C_1$-$C_8$, ou un atome d'hydrogène, de métal alcalin ou alcalino-terreux ou un radical dérivé d'amine, ou encore un radical de formule :

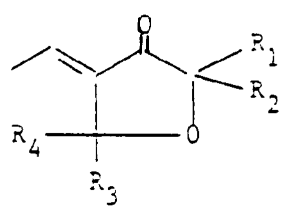

dans laquelle $R_1$, $R_2$, $R_3$ et $R_4$ ont les significations indiquées ci-dessus.

**13.** Composé selon la revendication 12, caractérisé par le fait qu'il est choisi parmi : la 5-(4'-hexyloxy benzylidène) 3-oxa 2,2,4, 4-tétraméthylcyclopentanone, la 5-(4'-allyloxy benzylidène) 3-oxa 2,2,4,4-tétraméthyl cyclopentanone, la 5-(4'-octyloxy benzylidène) 3-oxa 2,2,4,4-tétraméthyl cylopentanone, la 5-(3',4',5'-tribenzyloxy benzylidène) 3-oxa 2,2,4,4-tétraméthyl cyclopentanone, la 5-(4'-tert.-butoxy benzylidène) 3-oxa 2,2, 4,4-tétraméthyl cyclopentanone, la 5-(4'-méthoxy carbonyl benzylidène) 3-oxa 2,2,4,4- tétraméthyl cyclopentanone, la 5-(4'-n-butoxy 5'-méthoxy benzylidène) 3-oxa 2,2,4,4-tétraméthyl cyclopentanone, le 1,4-bis-[(2',2',4',4'-tétraméthyl-3 '-oxa-5'-oxo-1'-cyclopentylidène)méthyl] benzène, l'acide 4-[(2',2',4',4'-tétraméthyl-3'-oxa-5'-oxo-1'-cyclopentylidène)méthyl] benzoïque.

**14.** Procédé de préparation d'un composé de formule (I') selon la revendication 12 ou 13, caractérisé par le fait qu'il consiste à condenser un aldéhyde aromatique de formule (II') :

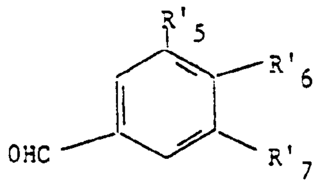

(II')

dans laquelle $R'_5$, $R'_6$, $R'_7$ ont les significations indiquées dans la revendication 12, sur une 3-oxa cyclopentanone de formule (III)

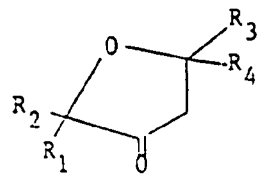

(III)

dans laquelle $R_1$, $R_2$ $R_3$ et $R_4$ ont les significations indiquées dans la revendication 12.

15. Procédé selon la revendication 14, caractérisé par le fait que la condensation de l'aldéhyde de formule (II') sur la cyclopentanone de formule (III) est effectuée en présence d'un alcoolate de métal alcalin, dans un solvant, à une température comprise entre -78°C et le point d'ébullition du solvant.

16. Procédé selon la revendication 14, caractérisé par le fait que la condensation de l'aldéhyde de formule (II') sur la cyclopentanone de formule (III) est effectuée en présence d'une base minérale, dans un solvant, à une température comprise entre 0°C et le point d'ébullition du mélange réactionnel.

17. Procédé selon la revendication 14, caractérisé par le fait que la condensation de l'aldéhyde de formule (II') sur la cyclopentanone de formule (III) est effectuée en présence d'un borane de formule :

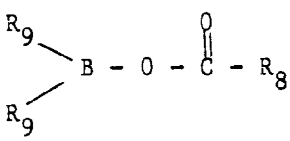

(IV)

dans laquelle $R_8$ représente un reste alkyle en $C_1$-$C_6$ et $R_9$ un reste alkyle en $C_1$-$C_4$ à une température d'environ 150°C, sans solvant.

## Patentansprüche

1. Kosmetische Zusammensetzung, dadurch **gekennzeichnet**, daß sie, in einem kosmetisch verträglichen Träger, als Verbindung, die die ultraviolette Strahlung von Wellenlängen von 280 bis 380 nm wegfiltert, eine wirksame Menge mindestens eines Derivats von 5-Benzyliden -3-oxacyclopentanon der allgemeinen Formel enthält:

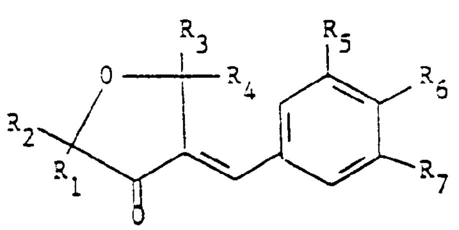

(I)

worin:
$R_1$, $R_2$, $R_3$ und $R_4$, gleich oder verschieden, einen linearen oder verzweigten $C_1$-$C_8$-Alkylrest, einen nicht-

substituierten oder durch Halogenatome oder $C_1$-$C_4$-Alkyl- oder - Alkoxygruppen substituierten Aralkyrest oder einen nicht-substituierten oder durch Halogenatome, $C_1$-$C_4$-Alkyl- oder - Alkoxyreste substuierten Arylrest darstellen oder auch $R_1$ und $R_2$ und/oder $R_3$ und $R_4$ mit dem Kohlenstoffatom, an das sie gebunden sind, einen gesättigten Ring mit 5 oder 6 Kohlenstoffatomen bilden;

$R_5$, $R_6$ und $R_7$, gleich oder verschieden, ein Wasserstoffatom, einen linearen oder verzweigten $C_1$-$C_8$-Alkylrest, einen linearen oder verzweigten $C_1$-$C_8$-Alkenylrest, einen linearen oder verzweigten $C_1$-$C_{12}$-Alkoxyrest, einen linearen oder verzweigten $C_2$-$C_8$-Alkenyloxyrest, einen linearen oder verzweigten $C_2$-$C_{12}$-Acyloxyrest, einen nicht-substituierten oder durch Halogenatome oder $C_1$-$C_4$-Alkyl- oder -Alkoxyreste substituierten Benzyloxyrest oder auch einen Rest -$COOR_{10}$ darstellen, worin $R_{10}$ ein Wasserstoffatom, einen linearen oder verzweigten $C_1$-$C_8$-Alkylrest oder ein Alkali-, Erdalkalimetall oder einen von einem Amin abgeleiteten Rest bedeutet; und worin einer der Substituenten $R_6$, $R_6$ und $R_7$ auch einen Rest der Formel bedeuten kann:

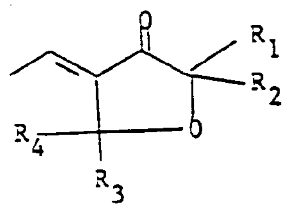

worin $R_1$, $R_2$, $R_3$ und $R_4$ die oben angegebenen Bedeutungen haben.

2. Kosmetische Zusammensetzung gemäß Asnpruch 1, dadurch **gekennzeichnet**, daß sie als Verbindung der Formel (I) mindestens eine der folgenden Verbindungen enthält:
5-Benzyliden-3-oxa-2,2,4,4-tetramethylcyclopentanon,
5-(4′-Hexyloxybenzyliden)-3-oxa-2,2,4,4-tetramethylcyclopentanon,
5-(4′-Allyloxybenzyliden)-3-oxa-2,2,4,4-tetramethylcyclopentanon,
5-(4′-Octyloxybenzyliden)-3-oxa-2,2,4,4-tetramethylcyclopentanon,
5-(3′,4′,5′-Tribenzyloxybenzyliden)-3-oxa-2,2,4,4-tetramethylcyclopentanon,
5-(4′-t-Butoxybenzyliden)-3-oxa-2,2,4,4-tetramethylcyclopentanon,
5-(4′-Methoxycarbonylbenzyliden)-3-oxa-2,2,4,4-tetramethylcyclopentanon,
5-(4′-n-Butoxy-5′-methoxybenzyliden)-3-oxa-2,2,4,4,tetramethylcyclopentanon,
1,4-Bis-[(2′,2′,4′,4′-tetramethyl-3′-oxa-5′-oxo-1′-cyclopentyliden)methyl]benzol,
4-[(2′,2′,4′,4′-Tetramethyl-3′-oxa-5′-oxo-1′-cyclopentyliden)methyl]benzoesäure.

3. Kosmetische Zusammensetzung gemäß Anspruch 1 oder 2, dadurch **gekennzeichnet**, daß sie in Form einer Lotion, Emulsion, eines Öls, Gels, Feststoffstäbchens oder Aerosols vorliegt.

4. Kosmetische Zusammensetzung gemäß jedem der Ansprüche 1 bis 3, dadurch **gekennzeichnet**, daß sie außerdem kosmetische Hilfsstoffe enthält, ausgewählt aus Verdickungsmitteln, Enthärtungsmitteln, Feuchtigkeitsmitteln, oberflächenaktiven Mitteln, Konservierungsmitteln, Antischaummitteln, Parfüms, Ölen, Wachsen, Lanolin, Monoalkoholen und Niedrigpolyolen, Treibmitteln, Färbemitteln und Pigmenten.

5. Kosmetische Zusammensetzung gemäß jedem der Ansprüche 1 bis 4, die in Form einer Zusammensetzung zum Schutz der menschlichen Haut vorliegt, dadurch **gekennzeichnet**, daß sie 0,25 bis 3 Gew.-% mindestens einer Verbindung der Formel (I) enthält.

6. Kosmetische Zusammensetzung gemäß jedem der Ansprüche 1 bis 4, die in Form einer Zusammensetzung gegen die Sonneneinwirkung vorliegt, dadurch **gekennzeichnet**, daß sie mindesten 0,5 bis 15 Gew.-% mindestens einer Verbindung der Formel (I) enthält.

7. Kosmetische Zusammensetzung gemäß Anspruch 6, dadurch **gekennzeichnet**, daß sie außerdem weitere Sonnenfilterstoffe enthält, die die UV-B- oder UV-A-Strahlen wegfiltern.

8. Kosmetische Zusammensetzung gemäß Anspruch 1 oder 2 zur Aufbringung auf die Haare, dadurch **gekennzeichnet**, daß sie in Form eines Shampoos, einer Lotion, eines Gels oder einer Emulsion zur Spülung, einer Frisur- oder Behandlungslotion oder eines -gels, einer Lotion oder eines Gels zum Bürsten oder der Wellengebung, eines Lacks für die Haare, einer Zusammensetzung für Dauerwelle, Färbung oder Entfärbung vorliegt und 0,25 bis 4 Gew.-% mindestens einer Verbindung der Formel (I) enthält.

**9.** Kosmetische Zusammensetzung gemäß Anspruch 1 oder 2, die in Form einer gefärbten oder nicht-gefärbten kosmetischen Zusammensetzung vorliegt, und welche gegen Lichteinwirkung stabilisiert ist, dadurch **gekennzeichnet**, daß sie zusammengesetzt ist aus einem Lack für die Haare, einer Lotion für die Wellengebung, einem Shampoo, Färbeshampoo, einer Färbezusammensetzung für die Haare oder einem Schminkprodukt, wie einem Nagellack, einem Lippenstift, einer Behandlungscreme für die Haut oder einer Teintgrundlage, und 0,25 bis 3 Gew.-% mindestens einer Verbindung der Formel (I) enthält.

**10.** Verfahren zur kosmetischen Behandlung der Haut und der natürlichen oder sensibilisierten Haare, um sie vor ultravioletter Strahlung von Wellenlängen von 280 bis 380 nm zu schützen, dadurch **gekennzeichnet**, daß man auf die Haut oder die Haare eine wirksame Menge einer kosmetischen Zusammensetzung, enthaltend mindestens eine Verbindung der Formel (I) gemäß Anspruch 1 oder 2, aufbringt.

**11.** Verfahren zum Schutz einer kosmetischen Zusammensetzung gegen ultraviolette Strahlen von Wellenlängen von 280 bis 380 nm, dadurch **gekennzeichnet**, daß man dieser Zusammensetzung eine wirksame Menge mindestens einer Verbindung der Formel (I) gemäß Anspruch 1 oder 2 einverleibt.

**12.** Derivat von 5-Benzyliden-3-oxacyclopentanon der allgemeinen Formel:

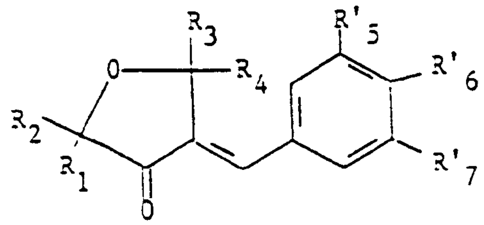

(I')

worin:

$R_1$, $R_2$, $R_3$ und $R_4$, gleich oder verschieden, einen linearen oder verzweigten $C_1$-$C_8$-Alkylrest, einen nicht-substituierten oder durch Halogenatome, $C_1$-$C_4$-Alkyl- oder -Alkoxyreste substituierten Aralkylrest oder einen nicht-substituierten oder durch Halogenatome, $C_1$-$C_4$-Alkyl- oder -Alkoxyreste substituierten Arylrest darstellen oder auch $R_1$ und $R_2$ und/oder $R_3$ und $R_4$ mit dem Kohlenstoffatom, an das sie gebunden sind, einen gesättigten Ring mit 5 oder 6 Kohlenstoffatomen bilden;

$R'_5$ und $R'_7$, gleich oder verschieden, ein Wasserstoffatom, einen linearen oder verzweigten $C_1$-$C_8$-Alkylrest, einen linearen oder verzweigten $C_2$-$C_8$-Alkenylrest, einen linearen oder verzweigen $C_1$-$C_{12}$-Alkoxyrest, einen linearen oder verzweigten $C_2$-$C_8$-Alkenyloxyrest, einen linearen oder verzweigten $C_2$-$C_{12}$-Acyloxyrest, einen nicht-substituierten oder durch Halogenatome, $C_1$-$C_4$-Alkyl- oder -Alkoxygruppen substituierten Benzyloxyrest oder einen -$COOR_{10}$-Rest darstellen, worin $R_{10}$ ein Wasserstoffatom, einen linearen oder verzweigten $C_1$-$C_8$-Alkylrest, ein Alkali-, Erdalkalimetall oder einen von einem Amin abgeleiteten Rest bedeutet;

und worin $R'_6$ einen linearen oder verzweigten $C_2$-$C_8$-Alkylrest, einen linearen oder verzweigten $C_2$-$C_8$-Alkenylrest, einen linearen oder verzweigten $C_2$-$C_{12}$-Alkoxyrest, einen linearen oder verzweigten $C_2$-$C_8$-Alkenyloxyrest, einen linearen oder verzweigten $C_2$-$C_{12}$-Acyloxyrest, einen nicht-substituierten oder durch Halogenatome, $C_1$-$C_4$-Alkyl- oder -Alkoxygruppen substituierten Benzyloxyrest, einen -$COOR_{10}$-Rest, worin $R_{10}$ einen linearen oder verzweigten $C_1$-$C_8$-Alkylrest, ein Wasserstoffatom, ein Alkali- oder Erdalkalimetallatom oder einen von einem Amin abgeleiteten Rest bedeutet, oder auch einen Rest der Formel darstellt:

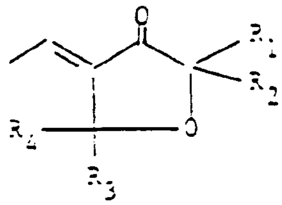

worin $R_1$, $R_2$, $R_3$ und $R_4$ die oben angegebenen Bedeutungen haben.

13. Verbindung gemäß Anspruch 12, dadurch **gekennzeichnet**, daß sie ausgewählt ist unter: 5-(4′-Hexyloxybenzyliden)-3-oxa-2,2,4,4-tetramethylcyclopentanon,
5-(4′-Allyloxybenzyliden)-3-oxa-2,2,4,4-tetramethylcyclopentanon,
5-(4′-Octyloxybenzyliden)-3-oxa-2,2,4,4-tetramethylcyclopentanon,
5-(3′,4′,5′-Tribenzyloxybenzyliden)-3-oxa-2,2,4,4-tetramethylcyclopentanon,
5-(4′-t-Butoxybenzyliden)-3-oxa-2,2,4,4-tetramethylcyclopentanon,
5-(4′-Methoxycarbonylbenzyliden)-3-oxa-2,2,4,4-tetramethylcyclopentanon,
5-(4′-n-Butoxy-5′-methoxybenzyliden)-3-oxa-2,2,4,4,tetramethylcyclopentanon,
1,4-Bis-[(2′,2′,4′,4′-tetramethyl-3′-oxy-5′-oxo-1′-cyclopentyliden)methyl]benzol,
4-[(2′,2′,4′,4′-Tetramethyl-3′-oxa-5′-oxo-1′-cyclopentyliden)methyl]benzoesäure.

14. Verfahren zur Herstellung der Formel (I′) gemäß Anspruch 12 oder 13, dadurch **gekennzeichnet**, daß man einen aromatischen Aldehyd der Formel (II′):

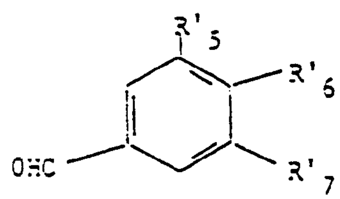

(II')

worin $R'_5$, $R'_6$ und $R'_7$ die in Anspruch 12 angegebenen Bedeutungen haben, mit einem 3-Oxacyclopentanon der Formel (III) kondensiert:

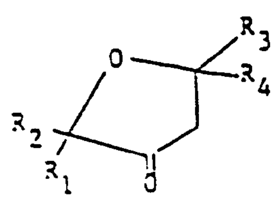

(III)

worin $R_1$, $R_2$, $R_3$ und $R_4$ die in Anspruch 12 angegebenen Bedeutungen haben.

15. Verfahren gemäß Anspruch 14, dadurch **gekennzeichnet**, daß die Kondensation des Aldehyds der Formel (II′) mit dem Cyclopentanon der Formel (III) in Gegenwart eines Alkalimetallalkoholats in einem Lösungsmittel bei einer Temperatur von -78°C bis zum Siedepunkt des Lösungsmittels durchgeführt wird.

16. Verfahren gemäß Anspruch 14, dadurch **gekennzeichnet**, daß die Kondensation des Aldehyds der Formel (II′) mit dem Cyclopentanon der Formel (III) in Gegenwart einer Mineralbase in einem Lösungsmittel bei einer Temperatur von 0°C bis zum Siedepunkt der Reaktionsmischung durchgeführt wird.

17. Verfahren gemäß Anspruch 14, dadurch **gekennzeichnet**, daß die Kondensation des Aldehyds der Formel (II′) mit dem Cyclopentanon der Formel (III) bei einer Temperatur von ca. 150°C ohne Lösungsmittel in Gegenwart eines Borans der Formel durchgeführt wird:

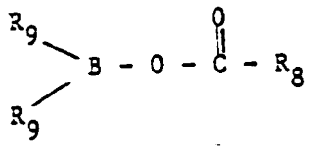

(IV)

worin $R_8$ einen $C_1$-$C_6$-Alkylrest und $R_9$ einen $C_1$-$C_4$-Alkylrest darstellen.

## Claims

1. Cosmetic composition, characterised in that it comprises, in a cosmetically acceptable substrate, as a compound which screens the ultraviolet radiation of wavelengths of between 280 and 380 nm, an effective quantity of at least one 5-benzylidene-3-oxacyclopentanone derivative of general formula :

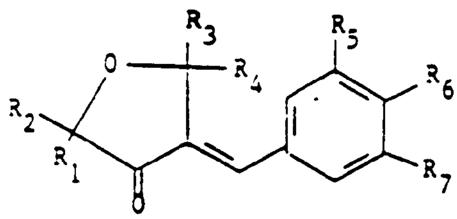

(I)

in which :

$R_1$, $R_2$, $R_3$ and $R_4$, which are identical or different, represent a $C_1$-$C_8$, linear or branched, alkyl residue, an aralkyl residue which is unsubstituted or substituted with halogen atoms or with $C_1$-$C_4$ alkyl or alkoxy residues, or an aryl residue which is unsubstituted or substituted with halogen atoms or $C_1$-$C_4$ alkyl or alkoxy residues, or else $R_1$ and $R_2$ and/or $R_3$ and $R_4$ form, with the carbon atom to which they are linked, a saturated ring containing 5 or 6 carbon atoms;

$R_5$, $R_6$ and $R_7$, which are identical or different, represent a hydrogen atom, a $C_1$-$C_8$, linear or branched, alkyl residue, a $C_2$-$C_8$, linear or branched, alkenyl residue, a $C_1$-$C_{12}$, linear or branched, alkoxy residue, a $C_2$-$C_8$, linear or branched, alkenyloxy residue, a $C_2$-$C_{12}$, linear or branched, acyloxy residue, a benzyloxy residue which is unsubstituted or substituted with halogen atoms or with $C_1$-$C_4$ alkyl or alkoxy residues, or a residue -$COOR_{10}$ where $R_{10}$ denotes a $C_1$-$C_8$, linear or branched, alkyl residue, a hydrogen atom, alkali or alkaline-earth metal atom or a residue derived from amine, it also being possible for one of the substituents $R_5$, $R_8$ and $R_7$ to represent a radical of formula :

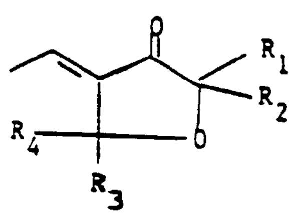

in which $R_1$, $R_2$, $R_3$ and $R_4$ have the meanings mentioned above.

2. Cosmetic composition according to Claim 1, characterised in that it comprises, as a compound of formula (I), at least one of the following compounds : 5-benzylidene-3-oxa-2,2,4,4-tetramethylcyclopentanone, 5-(4′-hexyloxybenzylidene)-3-oxa-2,2,4,4-tetramethylcyclopentanone, 5-(4′-allyloxybenzylidene)-3-oxa-2,2,4,4-tetramethylcyclopentanone, 5-(4′-octyloxybenzylidene)-3-oxa -2,2,4,4-tetramethylcyclopentanone, 5-(3′,4′,5′-tribenzyloxybenzylidene)-3-oxa-2,2,4,4-tetramethylcyclopentanone, 5-(4′-tert-butoxybenzylidene)-3-oxa-2,2,4,4-tetramethylcyclopentanone, 5-(4′-methoxycarbonylbenzylidene)-3-oxa-2,2,4,4-tetramethylcyclopentanone, 5-(4′-n-butoxy-5′-methoxybenzylidene)-3-oxa-2,2,4,4-tetramethylcyclopentanone, 1,4-bis [(2′,2′,4′,4′-tetramethyl-3′ -oxa-5′-oxo-1′-cyclopentylidene)methyl]benzene or 4-[(2′,2′,4′,4′-tetramethyl-3′-oxa-5′-oxo-1′-cyclopentylidene)methyl]benzoic acid.

3. Cosmetic composition according to Claim 1 or 2, characterised in that it is provided in the lotion, emulsion, oil, gel, solid stick or aerosol form.

4. Cosmetic composition according to any one of Claims 1 to 3, characterised in that it additionally contains cosmetic adjuvants chosen from thickeners, emollients, moisteners, surface active agents, preservatives, antifoaming agents, perfumes, oils, waxes, lanolin, lower mono-alcohols and polyols, propellants, dyes and pigments.

5. Cosmetic composition according to any one of Claims 1 to 4, which is provided in the form of a composition for protecting the human skin, characterised in that it contains 0.25 to 3 % by weight of at least one compound of formula (I).

6. Cosmetic composition according to any one of Claims 1 to 4, which is in the form of an antisun composition, characterised in that it contains 0.5 to 15 % by weight of at least one compound of formula (I).

7. Cosmetic composition according to Claim 6, characterised in that it additionally contains other sunscreens which screen the UV-B or UV-A rays.

8. Cosmetic composition according to Claim 1 or 2, intended to be applied to the hair, characterised in that it is in the form of a shampoo, lotion, gel or emulsion to be rinsed out, a styling or treatment lotion or gel, a lotion or gel for blow drying or hair setting, a hair lacquer or a perming, bleaching or dyeing composition and comprises 0.25 to 4 % by weight of at least one compound of formula (I).

9. Cosmetic composition according to Claim 1 or 2, which is provided in the form of a coloured or uncoloured cosmetic composition, stabilised against light, characterised in that it consists of a hair lacquer, a hair setting lotion, a shampoo, a colouring shampoo, a hair dyeing composition or a make-up product such as a nail varnish, a lipstick, a skin treatment cream or a foundation, and contains 0.25 to 3 % by weight of at least one compound of formula (I).

10. Process for cosmetic treatment of the skin and of natural or sensitive hair for the purpose of protecting them from ultraviolet radiation of wavelengths of between 280 and 380 nm, characterised in that it consists in applying to the skin or the hair an effective quantity of a cosmetic composition which contains at least one compound of formula (I) according to Claim 1 or 2.

11. Process for protecting a cosmetic composition against ultraviolet rays of wavelengths of between 280 and 380 nm, characterised in that it consists in incorporating, into this composition, an effective quantity of at least one compound of formula (I) according to Claim 1 or 2.

12. 5-Benzylidene-3-oxacyclopentanone derivative of general formula :

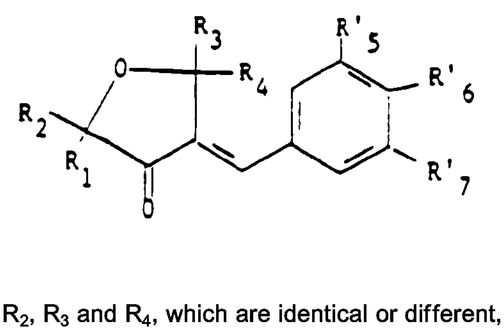

(I')

in which :

$R_1$, $R_2$, $R_3$ and $R_4$, which are identical or different, represent a $C_1$-$C_8$, linear or branched, alkyl residue, an aralkyl residue which is unsubstituted or substituted with halogen atoms or with $C_1$-$C_4$ alkyl or alkoxy residues, or an aryl residue which is unsubstituted or substituted with halogen atoms or $C_1$-$C_4$ alkyl or alkoxy residues, or else $R_1$ and $R_2$ and/or $R_3$ and $R_4$ form, with the carbon atom to which they are linked, a saturated ring containing 5 or 6 carbon atoms;

$R'_5$ and $R'_7$, which are identical or different, represent a hydrogen atom, a $C_1$-$C_8$, linear or branched, alkyl residue, a $C_2$-$C_8$, linear or branched, alkenyl residue, a $C_1$-$C_{12}$, linear or branched, alkoxy residue, a $C_2$-$C_8$, linear or branched alkenyloxy residue, a $C_2$-$C_{12}$, linear or branched, acyloxy residue, a benzyloxy residue which is unsubstituted or substituted with halogen atoms or $C_1$-$C_4$ alkyl or alkoxy groups, or a re-

sidue $-COOR_{10}$ where $R_{10}$ is a $C_1$-$C_8$, linear or branched, alkyl residue or denotes a hydrogen atom, alkali or alkaline-earth metal atom or a radical derived from amine;

$R'_6$ represents a $C_2$-$C_8$, linear or branched, alkyl residue, a $C_2$-$C_8$, linear or branched alkenyl residue, a $C_2$-$C_{12}$, linear or branched, alkoxy residue, a $C_2$-$C_8$, linear or branched, alkenyloxy residue, a $C_2$-$C_{12}$, linear or branched, acyloxy residue, a benzyloxy residue which is unsubstituted or substituted with halogen atoms or $C_1$-$C_4$ alkyl or alkoxy radicals or a residue $-COOR_{10}$ where $R_{10}$ denotes a $C_1$-$C_8$ alkyl radical, or a hydrogen atom, alkali or alkaline-earth metal atom or a radical derived from amine, or also a radical of formula :

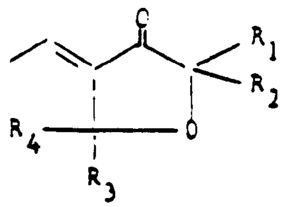

in which $R_1$, $R_2$, $R_3$ and $R_4$ have the meanings indicated above.

**13.** Compound according to Claim 12, characterised in that it is chosen from : 5-(4′-hexyloxybenzylidene)-3-oxa -2,2,4,4-tetramethylcyclopentanone, 5-(4′-allyloxybenzylidene)-3-oxa-2,2,4,4-tetramethylcyclopentanone, 5-(4′-octyloxybenzylidene)-3-oxa-2,2,4,4-tetramethylcyclopentanone, 5-(3′,4′,5′-tribenzyloxybenzylidene)-3-oxa-2 ,2,4,4-tetramethylcyclopentanone, 5-(4′-tert-butoxybenzylidene)-3-oxa-2,2,4,4-tetramethylcyclopentanone, 5-(4′-methoxycarbonylbenzylidene)-3-oxa-2,2,4,4-tetramethylcyclopentanone, 5-(4′-n-butoxy-5′-methoxybenzylidene)-3-oxa-2,2,4,4-tetramethylcyclopentanone, 1,4-bis-[(2′,2′,4′,4′-tetramethyl-3′-oxa-5′-oxo-1′-cyclopentylidene)methyl]benzene or 4-[(2′,2′,4′,4′-tetramethyl-3′-oxa-5′-oxo-1′-cyclopentylidene)methyl]benzoic acid.

**14.** Process for preparing a compound of formula (I′) according to Claim 12 or 13, characterised in that it consists in condensing an aromatic aldehyde of formula (II′) :

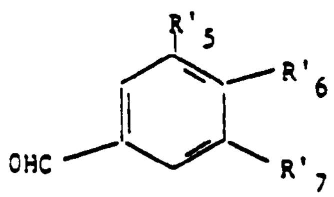

(II′)

in which $R'_5$, $R'_6$, $R'_7$ have the meanings indicated in Claim 12, with a 3-oxacyclopentanone of formula (III)

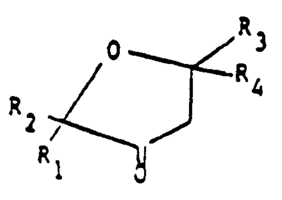

(III)

in which $R_1$, $R_2$, $R_3$ and $R_4$ have the meanings indicated in Claim 12.

**15.** Process according to Claim 14, characterised in that the condensation of the aldehyde of formula (II′) with the cyclopentanone of formula (III) is carried out in the presence of an alkali metal alcoholate, in a solvent, at a temperature of between -78°C and the boiling point of the solvent.

**16.** Process according to Claim 14, characterised in that the condensation of the aldehyde of formula (II′) with the cyclopentanone of formula (III) is carried out in the presence of an inorganic base, in a solvent, at a temperature of between 0°C and the boiling point of the reaction mixture.

**17.** Process according to Claim 14, characterised in that the condensation of the aldehyde of formula (II') with the cyclopentanone of formula (III) is carried out in the presence of a borane of formula :

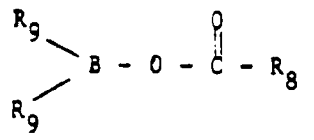

(IV)

in which $R_8$ represents a $C_1$-$C_6$ alkyl radical and $R_9$ a $C_1$-$C_4$ alkyl radical, at a temperature of approximately 150°C, without solvent.